Europäisches Patentamt

European Patent Office

Office européen des brevets

(11) Numéro de publication : **0 365 449 B1**

(12)    FASCICULE DE BREVET EUROPEEN

(45) Date de publication du fascicule du brevet :
26.05.93 Bulletin 93/21

(51) Int. Cl.⁵ : **G01N 33/543**

(21) Numéro de dépôt : **89430029.2**

(22) Date de dépôt : **16.10.89**

(54) **Immunoadsorbant à faible taux de relargage et son procédé d'obtention.**

(30) Priorité : **20.10.88 FR 8813776**

(43) Date de publication de la demande :
**25.04.90 Bulletin 90/17**

(45) Mention de la délivrance du brevet :
**26.05.93 Bulletin 93/21**

(84) Etats contractants désignés :
**AT BE CH DE ES FR GB GR IT LI LU NL SE**

(56) Documents cités :
**EP-A- 0 138 297**
**EP-A- 0 179 007**
**FR-A- 2 601 455**

(73) Titulaire : **IMMUNOTECH S.A.**
**70, route Léon Lachamp Case 915- Luminy**
**F-13288 Marseille Cédex 9 (FR)**

(72) Inventeur : **Delaage, Michel**
**16 rue Adolphe Thiers**
**F-13001 Marseille (FR)**
Inventeur : **Mejan, Odile**
**13 boulevard du Redon**
**F-13009 Marseille (FR)**
Inventeur : **Bourgois, Alain**
**123 Fc Traverse Parangon**
**F-13008 Marseille (FR)**

(74) Mandataire : **Rinuy, Santarelli**
**14, avenue de la Grande Armée**
**F-75017 Paris (FR)**

## Description

La présente invention concerne un immuno-adsorbant à faible taux de relargage, comportant un anticorps et un support chromatographique.

Un immunoadsorbant est défini comme étant un anticorps monoclonal ou polyclonal fixé directement ou indirectement à un support chromatographique solide du type résine ou gel et conservant la totalité ou la plus grande partie de son affinité initiale, avant liaison au support, pour un antigène correspondant.

L'affinité anticorps-antigène est à la fois très forte et selective et permet ainsi la capture sélective de l'antigène par mise en contact d'une solution diluée de ce dernier avec l'immunoadsorbant. Le rinçage au moyen d'une solution dissociante permet, en diminuant l'affinité de l'anticorps fixé au support pour l'antigène, la libération de ce dernier et sa récupération dans la solution dissociante.

Cependant, l'antigène ainsi obtenu n'est pas parfaitement pur car il contient toujours une proportion non négligeable de substances, notamment d'anticorps ou de fragments d'anticorps provenant de l'instabilité de l'immunoadsorbant. Cette instabilité se situe à trois niveaux :

- la fragilité du support lui-même, surtout lorsqu'il s'agit d'un gel de polysaccharide ;
- la fragilité de la liaison chimique entre le support et l'anticorps ;
- l'instabilité de l'anticorps lui-même, sensible aux agents protéolytiques présents dans la préparation à purifier et clivable en fragments $F_{ab}$ et $F_c$ ou même en domaines (Biochim. J. 1971 123, 538 ; Connell GE and Porter R.R., Biochemistry, 1973, <u>12</u>, 1130; Hochman J., Imber D. and Givol D.).

La contamination des effluents des colonnes d'immunopurification par les anticorps ou fragments de ceux-ci constitue un problème important. En effet, si les effluents sont destinés à un objectif analytique, l'anticorps ou ses fragments peuvent interférer dans les dosages de l'antigène qui présente alors des valeurs de concentration plus faibles que la réalité, du fait de la disparition d'une partie de l'antigène à doser par complexation du type anticorps-antigène.

De même, si les effluents doivent servir à la préparation d'un médicament, les anticorps ou fragments de ceux-ci sont considérés comme des impuretés immunogéniques de l'antigène purifié. Il convient alors de continuer la purification du mélange obtenu dans les effluents des colonnes d'immunopurification par l'utilisation d'une technique telle que filtration sur colonne échangeuse d'ions ou filtration sur une colonne protéine A-sépharose. Cette étape supplémentaire ne permet pas d'éliminer totalement les produits relargués et a l'inconvénient général de diminuer fortement le rendement en antigène d'une pureté suffisante pour être utilisé dans la fabrication d'un médicament.

Les résultats présentés dans la publication Thrombosis and Haemostasis 59(3), 364-371 (1988) montrent que le relargage en anticorps monoclonal (anti-vWF) peut être réduit en sélectionnant la méthode de couplage entre l'anticorps anti-vWF et le support chromatographique constitué par du Sephacryl S-1000. En effet, lorsque le Sephacryl S-1000 est activé à la benzoquinone selon la méthode de Brandt et al (Biochim. Biophys Acta 1975 ; 386 ; 196-202), le relargage en anticorps monoclonal anti-vWF est minimal mais atteint cependant environ 90 ng/cm³ d'immunoadsorbant (soit environ 75 ng/ml d'éluant).

Ainsi, bien que la méthode d'élution à la triéthanolamine permettrait une lyophilisation directe des éluants, la forte teneur en impuretés oblige la mise en oeuvre d'un procédé de purification supplémentaire afin d'obtenir un médicament d'une pureté suffisante.

Afin de remédier aux inconvénients rappelés ci-dessus, la Demanderesse s'est fixé comme but l'obtention d'un immunoadsorbant où le taux de relargage est considérablement réduit par rapport aux meilleurs résultats de l'art antérieur.

Ce but est atteint par l'objet selon la présente invention constitué par un immunoadsorbant à faible taux de relargage, comportant un anticorps et un support chromatographique, qui se caractérisé en ce qu'il comprend à l'échelle moléculaire :

a) des molécules d'anticorps, chacune d'entre elles liée de façon covalente à plusieurs chaînes latérales comportant chacune un radical biotinyle, au moins une de cesdites chaînes étant engagée dans une liaison d'affinité avec une molécule d'avidine liée de façon covalente audit support chromatographique, au moins une de cesdites chaînes étant libre et constituant un ligand potentiel du type biotine d'un site avidine d'une molécule du type avidine ;

b) des molécules d'avidine liées de façon covalente audit support chromatographique et comportant des sites libres, récepteurs potentiels de ligands du type biotine.

Les molécules d'avidine peuvent être d'origines biologiques diverses, notamment avidine de l'oeuf et streptavidine.

Ainsi, un immunoadsorbant selon la présente invention a, en plus de sa capacité de capture d'antigène correspondant à chaque molécule d'anticorps, une capacité de capture de haute affinité de molécules du type avidine et une capacité de capture de haute affinité de molécules du type biotine.

Ainsi, une solution comportant des molécules de biotine libre (non complexée par de l'avidine), est déplétée de sa biotine par contact avec un immunoadsorbant selon la présente invention.

De même, une solution comportant des molécules d'avidine libre (non complexée par de la biotine) est déplétée de son avidine par contact avec un immunoadsorbant selon la présente invention.

Cette double capacité intrinsèque de capture à la fois de la biotine et de l'avidine est une propriété générale de tout immunoadsorbant selon la présente invention.

Lorsque la solution entrant en contact avec l'immunoadsorbant selon l'invention ne contient pas des quantités saturantes en biotine ou avidine des sites actifs libres du type biotine ou avidine dudit immunoadsorbant, la double capacité de capture de molécules du type avidine et biotine s'exprime sur les constituants de l'immunoadsorbant et s'oppose ainsi au relargage dans les effluents de fragments de l'immunoadsorbant pouvant entrer dans une liaison de forte affinité du type avidine-biotine, notamment molécules ou fragments de molécules d'anticorps ayant une chaîne latérale comportant un radical biotinyle. Ceci a pour effet de réduire considérablement les taux de relargage de l'immunoadsorbant, aussi bien lors de la phase de capture de l'antigène que pendant le déblocage de celui-ci par une solution d'éluant dissociant.

Selon un autre aspect de l'invention, afin de se prémunir contre le relargage de fragments de l'antigène produits par protéolyse, le nombre moyen de chaînes latérales biotinylées par molécule d'anticorps sera compris entre 3 et 20 et de préférence entre 12 et 20, de façon à ce que ces chaines soient distribuées statistiquement sur toute la surface de l'anticorps et qu'il y en ait de préférence au moins une par domaine d'anticorps. Le calcul théorique suivant modélise un immunoadsorbant polybiotinylé selon l'invention où l'anticorps n'est pas fragmenté par des protéases.

L'immunoadsorbant placé dans une colonne est instable et libère l'anticorps avec la constante de vitesse $k'$. La contribution $dC$ à la concentration d'anticorps libéré, durant un intervalle de temps $dt$ est:

$$dC = k'.C_o.dt$$

où $C_o$ est la concentration d'anticorps dans l'immunoadsorbant et qu'on suppose constante car sa variation est négligeable. A la sortie de la colonne, cette contribution se trouve atténuée par la fraction $e^{-kt}$ où $k$ constante de réassociation, est la probabilité élémentaire de re-capture et $t$ la durée de transit (voir Proc. Natl. Acad. Sci. USA 1975 <u>72</u> 4840-4843 Denizot FC and Delaage M.A.).

La concentration $C_e$ éluée représente la somme de toutes les contributions élémentaires et en supposant la colonne illimitée :

$$C_e = k . C_o \int_{t=0}^{t=\infty} e^{-kt} . dt = \frac{k'}{k} . C_o$$

la constante de réassociation $k$ est une constante apparente du premier ordre qui s'écrit :

$$k = k_a.c_a$$

où $k_a$ est la constante de vitesse du deuxième ordre pour la réaction d'association avidine-biotine et $c_a$, la concentration en sites avidine libre. Il vient donc :

$$c_e = \frac{k'.C_o}{k_a.C_a}$$

On voit que la concentration $C_e$ éluée d'anticorps est proportionnelle à $\frac{Co}{Ca}$. Par conséquent, il sera avantageux d'augmenter le rapport R du nombre total de molécules d'avidine liées au support sur le nombre total des molécules d'anticorps biotinylées de façon à diminuer $\frac{C_o}{Ca}$

Ce rapport R sera de préférence d'environ 3 et plus.

Selon un autre aspect préféré de l'invention, l'immunoadsorbant aura un taux en anticorps polybiotinylé compris entre 0,1 et 5 mg par cm$^3$ d'immunoadsorbant.

La présente invention comporte également un procédé d'obtention d'un immunoadsorbant, caractérisé en ce qu'on effectue les opérations suivantes :

a) on fixe de manière covalente des molécules d'avidine à un support chromatographique en utilisant une méthode connue pour conserver aux molécules d'avidine ainsi greffées des sites libres, récepteurs potentiels de ligands du type biotine ;

b) on choisit un anticorps spécifique d'un antigène déterminé puis on fixe de façon covalente sur cet anticorps des chaînes contenant chacune un radical biotinyle de manière à obtenir un anticorps polybiotinylé;

c) on mélange, en présence éventuellement d'un solvant de dilution notamment du tampon phosphate isotonique (PBS), l'anticorps polybiotinylé obtenu en b) avec un excès du produit obtenu en a) de façon à ce qu'il reste des sites avidine libres, récepteurs potentiels de ligands du type biotine, notamment anticorps polybiotinylé ou ses fragments pourvus d'une chaîne biotinylée.

D'autres aspects et avantages de l'invention ressortiront de la description d'exemples qui va suivre, donnée à titre purement illustratif.

Exemple 1

Préparation d'un immunoadsorbant comportant un anticorps monoclonal anti-vWF polybiotinylé par plusieurs radicaux biotinyle et de l'avidine greffée sur un support chromatographique.

1. Préparation du support chromatographique avidine-Sephacryl.

Le Sephacryl® S-1000 (Pharmacia) est activé par la benzoquinone (Merck) selon la méthode de Brandt et coll. (Biochem. Biophys. Acta 1975, 386, 196-202).

Un volume de gel activé est incubé une nuit à 4°C sous agitation avec un volume d'une solution d'avidine (BioPartners, Bastogne) à 4 mg/ml dans du phosphate de sodium à pH 8.

Le gel est ensuite incubé 4 heures à temperature ambiante en tampon glycinate de sodium à pH 8,5. Puis il est placé successivement une heure en tampon d'acétate de sodium 0,1 M, NaCl 1 M, de pH 4 et une heure en tampon borate de sodium 0,02 M, NaCl 1 M, de pH 9. Finalement il est lavé avec du PBS et conservé dans 80 % de PBS mélangé à 20 % d'éthanol.

2. Détermination du nombre de sites de liaison pour la biotine sur le gel avidine-Sephacryl obtenu ci-dessus

Un dosage radio-immunologique par compétition est effectué comme suit :

100 µl d'une solution de PBS/BSA (sérum albumine bovine)/Tween® contenant 20 mg de gel avidine-Sephacryl® sont mélangés avec 100 µl d'une solution de $^3$H-biotine (Amersham) et 100 µl d'une solution de biotine froide de concentration connue (entre $10^{-8}$ et $10^{-7}$ mole/ ml) dans une solution PBS/BSA/Tween®.

Après une nuit d'incubation à température ambiante, le gel est lavé deux fois avec 1 ml de NaCl 0,15M. Les solutions de lavage sont prélevées et la radioactivité contenue dans les surnageants est évaluée avec un compteur en présence d'Instagel (Packard) comme liquide scintillant.

La fraction libre de $^3$H-biotine est exprimée en fonction de la quantité connue de biotine froide. Une droite est obtenue. Son intersection avec l'axe des abscisses permet de calculer le nombre maximal de sites libres, récepteurs potentiels d'un ligand du type biotine, du gel avidine-Sephacryl®. Ces sites correspondent à 3,2 mg d'avidine par ml du gel, soit 145 nanomoles de sites par ml du gel, au maximum.

3. Préparation de l'anticorps monoclonal anti-vWF humain.

L'anticorps choisi est anti-Facteur de von Willebrand humain (vWF) et provient d'un hybridome sélectionné après une série d'hybridations de cellules du myélome X63 de souris et de splénocytes de souris BALB/C immunisées avec le complexe FVIII/vWF selon les modes opératoires et de sélection décrits dans Thrombosis and Haemostasis 59(3), 364-371 (1988), O. Méjan et al, ou le brevet FR 84-14480 publié sous le N° 2.570.276.

L'anticorps retenu anti-vWF couplé directement à des particules solides gel de Sépharose® 4B ou Ultrogel® AcA22, a montré à la fois :
- une capacité à fixer le complexe FVIII/vWF ;
- une aptitude à relâcher le complexe FVIII/vWF en milieu alcalin (pH 8,7 à 10,4), les activités antihémophiliques A et Willebrand du complexe fixé puis relâché étant testées in vitro.

Cet anticorps retenu (AcM) par les tests précédents est purifié à partir du liquide d'ascite, par chromatographie d'affinité sur protéine A-Sépharose® puis dialysé toute une nuit à 4°C contre un tampon borate de sodium 0,02 M, pH 8,2. La concentration protéique est ajustée à 1 mg/ml.

La biotinylation est réalisée avec un excès molaire de 20 fois en ester biotinamidocaproate N-hydroxysuccinimide. Cet ester réactif est dissous dans du diméthylsulfoxyde (DMSO) pour obtenir une solution de 10 mg d'ester/ml de DMSO. Cette solution est ajoutée à la solution de AcM de concentration 1 mg/ml dans du tampon à pH 8,2 et le mélange est laissé à la température ambiante pendant 30 minutes. La réaction d'acylation des groupes amines des résidus lysine de l'anticorps est arrêtée par l'addition de NH$_4$Cl 1M, pH 7,5 en un rapport de 10 volumes pour un volume du mélange ci-dessus. Les chaînes latérales ainsi formées comportent chacune un radical biotinyle et sont chacune dans une liaison covalente amide avec un groupe amine de l'anticorps. L'excès de produit biotinylant est éliminé par dialyse contre du PBS.

Le rapport des densités optiques DO 280 nm/DO 260 nm doit être entre 1,5 et 1,7 pour le produit dialysé.

## 4. Préparation de l'immunoadsorbant

L'AcM polybiotinylé ci-dessus est mélangé une nuit à 4°C avec le gel avidine-Sephacryl® à raison de 0,8 mg d'AcM polybiotinylé par ml de gel. Le produit ainsi obtenu est ensuite incubé une heure à température ambiante en tampon pH 4 puis 9. Finalement, il est stocké dans un liquide contenant 80 % de PBS et 20 % d'éthanol.

Après immobilisation de l'AcM polybiotinylé, l'immunoadsorbant conserve $6,5.10^{-11}$ moles de sites avidine libres (récepteurs potentiels de ligands du type biotine) par mg d'immunoadsorbant (soit $6,5.10^{-8}$ moles de sites/ml d'immunoadsorbant).

## Résultats

L'immunoadsorbant précédent a une concentration en anticorps polybiotinylé de $5.10^{-6}$ M et une concentration $c_a$ en sites avidine de $6,5.10^{-5}$ M. On prépare avec celui-ci une colonne remplie sur 10 cm de hauteur et on élue cette colonne avec un éluant (tampon triéthanolamine 100 mM, pH 10) avec un flux de 80 ml/h/cm³. On observe une concentration en anticorps relargués de $0.2 \pm 0,1$ ng/ml d'éluant (soit $1,3.10^{-12} \pm 0,7$ M).

Dans un essai effectué parallèlement, on utilise une colonne de dimensions identiques remplie à la même hauteur par un immunoadsorbant de l'art antérieur ou le même anticorps anti-vWF est couplé directement au même support chromatographique (Sephacryl® S-1000) par la méthode à la benzoquinone (O. Méjan, publication ci-dessus référencée).

Ensuite, on élue avec le même éluant (tampon triéthanolamine 100 mM, pH 10). Le relargage en anticorps est alors de $4,8.10^{-10}$ M.

## Confrontation avec un modèle théorique

Les valeurs précédentes permettent d'évaluer la proportion k' d'anticorps détachés de l'immunoadsorbant de l'exemple 1 :

$$k' = \frac{4,8.10^{-10} \times 80}{5.10^{-6} \times 10 \times 3600 \text{ sec}} = 2,1.10^{-7} \text{ sec}^{-1}$$

Donc, pour une concentration en sites avidine de $6,5.10^{-5}$ M et une constante de vitesse d'association avidine-biotine de $k_a = 10^4$ M$^{-1}$.sec.$^{-1}$. Le relargage attendu est de :

$$c_e = \frac{2,1.10^{-7} \times 5.10^6 \times M}{6,5.10^{-5} \times 10^4} 1,6.10^{-12}M$$

La valeur expérimentale observée de $1,3.10^{-12}$M est donc très proche de cette valeur théorique.

Le temps de recapture $t_r$ est estimé à :

$$tr = \frac{\text{Log } 2}{k_a.c_a} = \frac{0,7 \times \text{sec}}{6,5.\,10^{-5} \times 10^4} = 1,1 \text{ sec}$$

Le temps de transit observé sur la colonne expérimentale ci-dessus est de 450 sec et l'approximation, qui a consisté à considérer dans les calculs précédents la colonne comme illimitée, trouve ici sa justification.

## Démonstration d'un phénomène de stabilité dynamique

L'AcM ou ses fragments et domaines qui se détachent de l'édifice moléculaire de l'immunoadsorbant peuvent être récupérés par les molécules d'avidine liées par liaison covalente au support, dans la mesure ou l'AcM, ses fragments ou domaines sont eux-mêmes pourvus d'une chaîne biotinylée. La stabilité apparente du gel est alors une stabilité dynamique.

Par ailleurs, les molécules d'avidine peuvent créer, par l'intermédiaire des radicaux biotinyle des chaînes latérales fixées de manière covalente à l'AcM, un réseau tridimensionnel qui stabilise la liaison de l'AcM au support chromatographique.

Afin d'évaluer la part respective de ces deux phénomène, les sites résiduels avidine de l'immunoadsorbant de l'exemple 1 ont été saturés par de la biotine et on a contrôlé le relargage en AcM et fragments par une technique ELISA, en sandwich spécifique des chaînes H et L d'immunoglobulines de souris.

Dans ces conditions d'élution (tampon triéthanolamine, pH 10) un relargage de $1,2.10^{-10}$M a été observé, proche de la valeur trouvée de $4,8.10^{-10}$M pour un immunoadsorbant témoin où le même anticorps (non polybiotinylé) est fixé directement sur du Sephacryl® S-1000 activé à la benzoquinone.

Il a été indiqué ci-dessus que l'immunoadsorbant de l'exemple 1, relargue de l'anticorps dans le même éluant à une concentration de $1,3.10^{-12} \pm 0,7M$.

Ainsi, la saturation par de la biotine des sites libres des molécules d'avidine liées de façon covalente au support a bien supprimé la stabilité dynamique qui représente l'essentiel de la stabilisation de l'AcM dans l'immunoadsorbant selon la présente invention.

Exemple 2

Utilisation de l'immunoadsorbant de l'exemple 1 pour l'immunopurification du complexe facteur VIII/vWF humain à partir du plasma.

Cet immunoadsorbant permet d'obtenir un complexe FVIII/vWF ayant une contamination maximale de 10 ng d'AcM murins pour 100 U.I. de FVIII du complexe immunopurifié à 2 U.I./ml

Dans le cadre d'un traitement antihémophilique A chez l'homme, ces quantités semblent pouvoir être injectées sans danger d'après Hrinda et coll. Symposium international INSERM "Biotechnologie des protéines du plasma" Nancy 17-19 mai 1988 - Communication orale : Monoclonal antibody purification of plasma proteins removes viral contaminants.

Exemple 3

1° Préparation d'un immunoadsorbant comportant un anticorps monoclonal anti-hFSH ("Follicule stimulating hormone") polybiotinylé et de l'avidine greffée sur un support chromatographique.

L'anticorps monoclonal utilisé est un anti-hFSH (N° 32621-Benkirane et al, J. Immunol. Meth., 1987, 98 pp. 173-181).

Cet anticorps est polybiotinylé par de l'ester biotinamidocaproate N-hydroxysuccinimide à raison de 8 % en poids d'ester par rapport au poids de l'anticorps par mélange durant 15 minutes sous agitation modérée.

La réaction d'acylation est bloquée par l'ajout d'une solution aqueuse 10 mM en $NH_4Cl$. La solution résultante est dialysée contre du PBS.

Le gel Séphacryl® S-1000 avidiné comme dans l'exemple 1 est lavé avec 5 volumes de NaCl 0,15 M, puis on mélange l'anticorps anti-hFSH polybiotinylé avec le gel à raison de 1 mg d'anticorps par ml de gel pendant 18 à 24 heures, à 4°C, sous agitation rotative. Le produit ainsi obtenu est lavé avec 5 volumes d'une solution de NaCl 0,15 M, puis avec 5 volumes d'une solution acétate de sodium 1M, NaCl 1M à pH 4 avant d'être équilibré et stocké dans une solution de NaCl 0, 15 M contenant 10 mM d'azoture de sodium ou dans une solution de PBS 80 % et d'éthanol 20 % à pH 7,2.

2° Utilisation pour une immuno-épuration et l'obtention d'un sérum zéro en h FSH.

On prépare une colonne d'environ 100 ml chargée de l'immunoadsorbant ci-dessus (100 mg d'anticorps) et l'on fait passer 10 litres de sérum à 100 ml/heure.

La colonne est à température ambiante. Le réservoir de sérum à éluer et les fractions récoltées sont maintenus à 4°C (volume de chaque fraction : 1 litre).

Les dix fractions récoltées sont chacune testées en h FSH en RIA avec et sans surcharge. La concentration résiduelle en h FSH est indétectable (inférieure à 0.1 U.I./litre de sérum épuré, ce qui correspond à une épuration d'au moins 99,9 %).

D'autres essais ont été effectués en remplaçant l'ester biotinamidocaproate N-hydroxysuccinimide par l'ester d-biotinyl-N-hydroxysuccinimide en opérant selon le mode préparatoire de l'exemple 1. On obtient un anticorps anti-vWF polybiotinylé. Le nombre moyen de molécules de biotine couplées à chaque molécule d'AcM est déterminé en utilisant de l'ester $^3$H-d-biotinyl-N-hydroxysuccinimide (Amersham) à 10.000 cpm par ml et en mesurant la radioactivité associée à l'AcM ainsi polybiotinylé. Les mesures donnent 12 chaînes latérales comportant chacune un radical biotinyle, par molécule d'AcM.

Enfin, d'autres essais ont montré qu'en changeant les conditions de l'acylation de l'AcM par l'ester réactif ci-dessus, il était possible de greffer en moyenne de 3 à 20 chaînes biotinylées sur chaque molécule d'AcM.

Il va de soi que la présente invention n'a été décrite qu'à titre purement explicatif et nullement limitatif et que toute modification notamment au niveau des équivalences pourra y être apportée, sans sortir de son cadre.

## Revendications

**Revendications pour les Etats contractants suivants : AT, BE, CH, DE, FR, GB, GR, IT, LI, LU, NL, SE**

**1.** Immunoadsorbant à faible taux de relargage comportant un anticorps et un support chromatographique, caractérisé en ce qu'il comprend, à l'échelle moléculaire :
(a) des molécules d'anticorps, chacune d'entre elles liée de façon covalente à plusieurs chaînes latérales comportant chacune un radical biotinyle, au moins une de cesdites chaînes étant engagée dans une liaison d'affinité avec une molécule d'avidine liée de façon covalente audit support chromatographique, au moins une de ces dites chaînes étant libre et constituant un ligand potentiel du type biotine d'un site avidine d'une molécule du type avidine ;
(b) des molécules d'avidine liées de façon covalente audit support chromatographique et comportant des sites libres, récepteurs potentiels de ligands du type biotine.

**2.** Immunoadsorbant selon la revendication 1, caractérisé en outre en ce que l'anticorps est monoclonal.

**3.** Immunoadsorbant selon la revendication 2, caractérisé en ce que l'anticorps monoclonal est anti-vWF et provient d'un hybridome sélectionné après une série d'hybridations de cellules de myélome X63 de souris et de splénocytes de souris BALB/C immunisées avec le complexe FVIII/vWF humain.

**4.** Immunoadsorbant selon la revendication 2, caractérisé en ce que l'anticorps monoclonal est anti-h FSH.

**5.** Immunoadsorbant selon l'une quelconque des revendications précédentes, caractérisé en ce que ledit support chromatographique est un gel du type Séphacryl®.

**6.** Immunoadsorbant selon l'une quelconque des revendications précédentes, caractérisé en ce que le rapport du nombre total des molécules d'avidine liées au support sur le nombre total des molécules d'anticorps est d'environ 3 et plus.

**7.** Immunoadsorbant selon l'une quelconque des revendications précédentes, caractérisé en ce que le nombre moyen de chaînes latérales biotinylées par molécule d'anticorps est compris entre 3 et 20.

**8.** Immunoadsorbant selon la revendication 7, caractérisé en ce que ledit nombre moyen est compris entre 12 et 20.

**9.** Immunoadsorbant selon l'une quelconque des revendications précédentes, caractérisé en ce que le taux en anticorps polybiotinylé est compris entre 0,1 et 5 mg par $cm^3$ d'immunoadsorbant.

**10.** Procédé d'obtention d'un immunoadsorbant, caractérisé en ce qu'on effectue les opérations suivantes :
(a) on fixe de manière covalente des molécules d'avidine à un support chromatographique en utilisant une méthode connue pour conserver aux molécules d'avidine ainsi greffées des sites libres, récepteurs potentiels de ligands du type biotine ;
(b) on choisit un anticorps spécifique d'un antigène déterminé puis on fixe de façon covalente sur cet anticorps des chaînes contenant chacune un radical biotinyle de manière à obtenir un anticorps polybiotinylé;
(c) on mélange, en présence éventuellement d'un solvant de dilution notamment du tampon phosphate isotonique (PBS), l'anticorps polybiotinylé obtenu en (b) avec un excès du produit obtenu en a) de façon à ce qu'il reste des sites avidine libres, récepteurs potentiels de ligands du type biotine, notamment anticorps polybiotinylé ou ses fragments pourvus d'une chaîne biotinylée.

**Revendications pour l'Etat contractant suivant : ES**

**1.** Immunoadsorbant à faible taux de relargage comportant un anticorps et un support chromatographique, caractérisé en ce qu'il comprend, à l'échelle moléculaire :
(a) des molécules d'anticorps, chacune d'entre elles liée de façon covalente à plusieurs chaînes latérales comportant chacune un radical biotinyle, au moins une de cesdites chaînes étant engagée dans un liaison d'affinité avec une molécule d'avidine liée de façon covalente audit support chromatographique, au moins une de ces dites chaînes étant libre et constituant un ligand potentiel du type biotine d'un site avidine d'une molécule du type avidine ;

(b) des molécules d'avidine liées de façon covalente audit support chromatographique et comportant des sites libres, récepteurs potentiels de ligands du type biotine.

2. Immunoadsorbant selon la revendication 1 , caractérisé en outre en ce que l'anticorps est monoclonal.

3. Immunoadsorbant selon la revendication 2, caractérisé en ce que l'anticorps monoclonal est anti-vWF et provient d'un hybridome sélectionné après une série d'hybridations de cellules de myélome X63 de souris et de splénocytes de souris BALB/C immunisées avec le complexe FVIII/vWF humain.

4. Immunoadsorbant selon la revendication 2, caractérisé en ce que l'anticorps monoclonal est anti-hFSH.

5. Immunoadsorbant selon l'une quelconque des revendications précédentes, caractérisé en ce que ledit support chromatographique est un gel du type Séphacryl®.

6. Immunoadsorbant selon l'une quelconque des revendications précédentes, caractérisé en ce que le rapport du nombre total des molécules d'avidine liées au support sur le nombre total des molécules d'anticorps est d'environ 3 et plus.

7. Immunoadsorbant selon l'une quelconque des revendications précédentes, caractérisé en ce que le nombre moyen de chaînes latérales biotinylées par molécule d'anticorps est compris entre 3 et 20.

8. Immunoadsorbant selon la revendication 7, caractérisé en ce que ledit nombre moyen est compris entre 12 et 20.

9. Immunoadsorbant selon l'une quelconque des revendications précédentes, caractérisé en ce que le taux en anticorps polybiotinylé est compris entre 0,1 et 5 mg par $cm^3$ d'immunoadsorbant.

10. Procédé d'obtention d'un immunoadsorbant à faible taux de relargage comportant un anticorps et un support chromatographique comprenant, à l'échelle moléculaire :

(a) des molécules d'anticorps, chacune d'entre elles liée de façon covalente à plusieurs chaînes latérales comportant chacune un radical biotinyle, au moins une de cesdites chaînes étant engagée dans un liaison d'affinité avec une molécule d'avidine liée de façon covalente audit support chromatographique, au moins une de ces dites chaînes étant libre et constituant un ligand potentiel du type biotine d'un site avidine d'une molécule du type avidine ;

(b) des molécules d'avidine liées de façon covalente audit support chromatographique et comportant des sites libres, récepteurs potentiels de ligands du type biotine dans lequel l'on effectue les opérations suivantes :

(a) on fixe de manière covalente des molécules d'avidine à un support chromatographique en utilisant une méthode connue pour conserver aux molécules d'avidine ainsi greffées des sites libres, récepteurs potentiels de ligands du type biotine ;

(b) on choisit un anticorps spécifique d'un antigène déterminé puis on fixe de façon covalente sur cet anticorps des chaînes contenant chacune un radical biotinyle de manière à obtenir un anticorps polybiotinylé ;

(c) on mélange, en présence éventuellement d'un solvant de dilution notamment du tampon phosphate isotonique (PBS), l'anticorps polybiotinylé obtenu en (b) avec un excès du produit obtenu en a) de façon à ce qu'il reste des sites avidine libres, récepteurs potentiels de ligands du type biotine, notamment anticorps polybiotinylé ou ses fragments pourvus d'une chaîne biotinylée.

11. Procédé selon la revendication 10, caractérisé en ce que l'anticorps est monoclonal.

12. Procédé selon la revendication 11, caractérisé en ce que l'anticorps monoclonal est anti-vWF et provient d'un hybridome sélectionné après une série d'hybridations de cellules de myélome X63 de souris et de splénocytes de souris BALB/C immunisées avec le complexe FVIII/vWF humain.

13. Procédé selon la revendication 11, caractérisé en ce que l'anticorps monoclonal est anti-hFSH.

14. Procédé selon l'une quelconque des revendications 10 à 13, caractérisé en ce que ledit support chromatographique est un gel du type Séphacryl®.

15. Procédé selon l'une quelconque des revendications 10 à 14, caractérisé en ce que le rapport du nombre total des molécules d'avidine liées au support sur le nombre total des molécules d'anticorps est d'environ 3 et plus.

16. Procédé selon l'une quelconque des revendications 10 à 15, caractérisé en ce que le nombre moyen de chaînes latérales biotinylées par molécule d'anticorps est compris entre 3 et 20.

17. Procédé selon la revendication 16, caractérisé en ce que ledit nombre moyen est compris entre 12 et 20.

18. Procédé selon l'une quelconque des revendications 10 à 17, caractérisé en ce que le taux en anticorps polybiotinylé est compris entre 0,1 et 5 mg par cm³ d'immunoadsorbant.


**Patentansprüche**

**Patentansprüche für folgende Vertragsstaaten : AT, BE, CH, DE, FR, GB, GR, IT, LI, LU, NL, SE**

1. Immunoadsorbens mit geringer Freisetzungsrate, umfassend einen Antikörper und einen chromatografischen Träger, dadurch gekennzeichnet, daß es auf Molbasis folgendes enthält:
   (a) Antikörpermoleküle, von denen jedes auf kovalente Weise an mehrere Seitenketten mit jeweils einem Biotinylrest gebunden ist, wobei mindestens eine dieser Ketten eine Affinitätsbindung mit einem Avidinmolekül einnimmt, welches auf kovalente Weise an den chromatografischen Träger gebunden ist, und wobei mindestens eine dieser Ketten frei ist und einen potentiellen Liganden vom Biotintyp einer Avidinstelle eines Moleküls vom Avidintyp bildet;
   (b) Avidinmoleküle, die auf kovalente Weise an den chromatografischen Träger gebunden sind und freie Stellen, nämlich potentielle Rezeptoren von Liganden vom Biotintyp, aufweisen.

2. Immunoadsorbens nach Anspruch 1, weiters dadurch gekennzeichnet, daß der Antikörper monoklonal ist.

3. Immunoadsorbens nach Anspruch 2, dadurch gekennzeichnet, daß der monoklonale Antikörper ein anti-vWF-Antikörper ist und von einem Hybridom stammt, welches nach einer Reihe von Hybridisierungen von mit dem FVIII/menschlicher vWF-Komplex immunisierten Myelomzellen X63 der Maus und Splenozyten der Maus BALB/C selektioniert wurde.

4. Immunoadsorbens nach Anspruch 2, dadurch gekennzeichnet, daß der monoklonale Antikörper ein anti-hFSH-Antikörper ist.

5. Immunoadsorbens nach einem der vorhergehenden Ansprüche, dadurch gekennzeichnet, daß der chromatografische Träger ein Gel vom Typ Sephacryl® ist.

6. Immunoadsorbens nach einem der vorhergehenden Ansprüche, dadurch gekennzeichnet, daß das Verhältnis der Gesamtanzahl der an den Träger gebundenen Avidinmoleküle zur Gesamtanzahl der Antikörpermoleküle etwa 3 und mehr beträgt.

7. Immunoadsorbens nach einem der vorhergehenden Ansprüche, dadurch gekennzeichnet, daß die mittlere Anzahl an Biotinyl-Seitenketten pro Molekül Antikörper zwischen 3 und 20 beträgt.

8. Immunoadsorbens nach Anspruch 7, dadurch gekennzeichnet, daß die mittlere Anzahl zwischen 12 und 20 beträgt.

9. Immunoadsorbens nach einem der vorhergehenden Ansprüche, dadurch gekennzeichnet, daß der Gehalt an Polybiotinyl-Antikörpern zwischen 0,1 und 5 mg pro cm³ Immunoadsorbens beträgt.

10. Verfahren zur Herstellung eines Immunoadsorbens, dadurch gekennzeichnet, daß man die folgenden Verfahrensschritte ausführt:
    (a) man fixiert auf kovalente Weise Avidinmoleküle an einem chromatografischen Träger unter Anwendung einer bekannten Methode derart, daß an den so aufgepfropften Avidinmolekülen freie Stellen, nämlich potentielle Rezeptoren von Liganden vom Biotintyp, bestehen bleiben;
    (b) man wählt einen spezifischen Antikörper eines bestimmten Antigens, dann fixiert man auf kovalente Weise die jeweils einen Biotinylrest enthaltenden Ketten an diesen Antikörper derart, daß ein Polybiotinyl-Antikörper erhalten wird;

(c) man mischt, gegebenenfalls in Gegenwart eines Verdünnungslösungsmittels, insbesondere eines isotonischen Phosphatpuffers (PBS), den in (b) erhaltenen Polybiotinyl-Antikörper mit einem Überschuß des in (a) erhaltenen Produkts derart, daß freie Avidinstellen, nämlich potentielle Rezeptoren von Liganden vom Biotintyp, verbleiben, insbesondere den Polybiotinyl-Antikörper oder mit einer Biotinylkette versehene Fragmente desselben.

**Patentansprüche für folgenden Vertragsstaat : ES**

1. Immunoadsorbens mit geringer Freisetzungsrate, umfassend einen Antikörper und einen chromatografischen Träger, dadurch gekennzeichnet, daß es auf Molbasis folgendes enthält:

(a) Antikörpermoleküle, von denen jedes auf kovalente Weise an mehrere Seitenketten mit jeweils einem Biotinylrest gebunden ist, wobei mindestens eine dieser Ketten eine Affinitätsbindung mit einem Avidinmolekül einnimmt, welches auf kovalente Weise an den chromatografischen Träger gebunden ist, und wobei mindestens eine dieser Ketten frei ist und einen potentiellen Liganden vom Biotintyp einer Avidinstelle eines Moleküls vom Avidintyp bildet;

(b) Avidinmoleküle, die auf kovalente Weise an den chromatografischen Träger gebunden sind und freie Stellen, nämlich potentielle Rezeptoren von Liganden vom Biotintyp, aufweisen.

2. Immunoadsorbens nach Anspruch 1, weiters dadurch gekennzeichnet, daß der Antikörper monoklonal ist.

3. Immunoadsorbens nach Anspruch 2, dadurch gekennzeichnet, daß der monoklonale Antikörper ein anti-vWF-Antikörper ist und von einem Hybridom stammt, welches nach einer Reihe von Hybridisierungen von mit dem FVIII/menschlicher vWF-Komplex immunisierten Myelomzellen X63 der Maus und Splenozyten der Maus BALB/C selektioniert wurde.

4. Immunoadsorbens nach Anspruch 2, dadurch gekennzeichnet, daß der monoklonale Antikörper ein anti-hFSH-Antikörper ist.

5. Immunoadsorbens nach einem der vorhergehenden Ansprüche, dadurch gekennzeichnet, daß der chromatografische Träger ein Gel vom Typ Sephacryl® ist.

6. Immunoadsorbens nach einem der vorhergehenden Ansprüche, dadurch gekennzeichnet, daß das Verhältnis der Gesamtanzahl der an den Träger gebundenen Avidinmoleküle zur Gesamtanzahl der Antikörpermoleküle etwa 3 und mehr beträgt.

7. Immunoadsorbens nach einem der vorhergehenden Ansprüche, dadurch gekennzeichnet, daß die mittlere Anzahl an Biotinyl-Seitenketten pro Molekül Antikörper zwischen 3 und 20 beträgt.

8. Immunoadsorbens nach Anspruch 7, dadurch gekennzeichnet, daß die mittlere Anzahl zwischen 12 und 20 beträgt.

9. Immunoadsorbens nach einem der vorhergehenden Ansprüche, dadurch gekennzeichnet, daß der Gehalt an Polybiotinyl-Antikörpern zwischen 0,1 und 5 mg pro cm$^3$ Immunoadsorbens beträgt.

10. Verfahren zur Herstellung eines Immunoadsorbens mit geringer Freisetzungsrate, umfassend einen Antikörper und einen chromatografischen Träger, welches auf Molbasis:

(a) Antikörpermoleküle, von denen jedes auf kovalente Weise an mehrere Seitenketten mit jeweils einem Biotinylrest gebunden ist, wobei mindestens eine dieser Ketten eine Affinitätsbindung mit einem Avidinmolekül einnimmt, welches auf kovalente Weise an den chromatografischen Träger gebunden ist, und wobei mindestens eine dieser Ketten frei ist und einen potentiellen Liganden vom Biotintyp einer Avidinstelle eines Moleküls vom Avidintyp bildet;

(b) Avidinmoleküle, die auf kovalente Weise an den chromatografischen Träger gebunden sind und freie Stellen, nämlich potentielle Rezeptoren der Liganden vom Biotintyp aufweisen, enthält,

wobei man die folgenden Verfahrensschritte ausführt:

(a) man fixiert auf kovalente Weise Avidinmoleküle an einem chromatografischen Träger unter Anwendung einer bekannten Methode derart, daß an den so aufgepfropften Avidinmolekülen freie Stellen, nämlich potentielle Rezeptoren von Liganden vom Biotintyp, verbleiben;

(b) man wählt einen spezifischen Antikörper eines bestimmten Antigens, dann fixiert man auf kovalente Weise die jeweils einen Biotinylrest enthaltenden Ketten an diesen Antikörper derart, daß ein Polybiotinyl-Antikörper erhalten wird;

(c) man mischt, gegebenenfalls in Gegenwart eines Verdünnungslösungsmittels, insbesondere eines isotonischen Phosphatpuffers (PBS), den in (b) erhaltenen Polybiotinyl-Antikörper mit einem Überschuß des in (a) erhaltenen Produkts derart, daß freie Avidinstellen verbleiben, nämlich potentielle Rezeptoren von Liganden vom Biotintyp, insbesondere Polybiotinyl-Antikörper oder mit einer Biotinylkette versehene Fragmente derselben.

11. Verfahren nach Anspruch 10, dadurch gekennzeichnet, daß der Antikörper monoklonal ist.

12. Verfahren nach Anspruch 11, dadurch gekennzeichnet, daß der monoklonale Antikörper ein anti-vWF-Antikörper ist und von einem Hybridom stammt, welches nach einer Reihe von Hybridisierungen von mit dem FVIII/menschlicher vWF-Komplex immunisierten Myelomzellen X63 der Maus und Splenozyten der Maus BALB/C selektiert wurde.

13. Verfahren nach Anspruch 11, dadurch gekennzeichnet, daß der monoklonale Antikörper ein anti-hFSH-Antikörper ist.

14. Verfahren nach einem der Ansprüche 10 bis 13, dadurch gekennzeichnet, daß der chromatografische Träger ein Gel vom Typ Sephacryl® ist.

15. Verfahren nach einem der Ansprüche 10 bis 14, dadurch gekennzeichnet, daß das Verhältnis der Gesamtanzahl der an den Träger gebundenen Avidinmoleküle zur Gesamtanzahl der Antikörpermoleküle etwa 3 und mehr beträgt.

16. Verfahren nach einem der Ansprüche 10 bis 15, dadurch gekennzeichnet, daß die mittlere Anzahl an Biotinyl-Seitenketten pro Molekül Antikörper zwischen 3 und 20 beträgt.

17. Verfahren nach Anspruch 16, dadurch gekennzeichnet, daß die mittlere Anzahl zwischen 12 und 20 beträgt.

18. Verfahren nach einem der Ansprüche 10 bis 17, dadurch gekennzeichnet, daß der Gehalt an Polybiotinyl-Antikörpern zwischen 0,1 und 5 mg pro cm$^3$ Immunoadsorbens beträgt.

## Claims

### Claims for the following Contracting States : AT, BE, CH, DE, FR, GB, GR, IT, LI, LU, NL, SE

1. Immunoadsorbent with a low rate of release comprising an antibody and a chromatographic support, characterized in that it contains, on the molecular level:

   (a) antibody molecules, each of them bound covalently to several side chains each containing a biotinyl radical, at least one of these said chains being engaged in an affinity bonding with an avidin molecule bound covalently to the said chromatographic support, at least one of these said chains being free and constituting a potential ligand of biotin type for an avidin site of a molecule of avidin type;

   (b) avidin molecules bound covalently to the said chromatographic support and containing free sites, potential receivers of ligands of biotin type.

2. Immunoadsorbent according to claim 1, characterized in addition in that the antibody is monoclonal.

3. Immunoadsorbent according to claim 2, characterized in that the monoclonal antibody is anti-vWF and results from a hybridoma selected after a series of hybridizations of mouse X63 myeloma cells and of BALB/C mouse splenocytes immunized with the FVIII/vWF human complex.

4. Immunoadsorbent according to claim 2, characterized in that the monoclonal antibody is anti-hFSH.

5. Immunoadsorbent according to any one of the previous claims, characterized in that the said chromatographic support is a gel of Sephacryl $^R$ type.

6. Immunoadsorbent according to any one of the previous claims, characterized in that the ratio of the total number of avidin molecules bound to the support to the total number of antibody molecules is about 3 or more.

7. Immunoadsorbent according to any one of the previous claims, characterized in that the average number of biotinylated side chains per antibody molecule is comprised between 3 and 20.

8. Immunoadsorbent according to claim 7, characterized in that the said average number is comprised between 12 and 20.

9. Immunoadsorbent according to any one of the previous claims, characterized in that the amount of polybiotinylated antibody is comprised between 0.1 and 5 mg per cm$^3$ of immunoadsorbent.

10. Process for obtaining an immunoadsorbent, characterized in that the following operations are carried out:
(a) avidin molecules are fixed covalently to a chromatographic support using a known method for preserving free sites, which are potential receivers of ligands of biotin type, for the avidin molecules thus grafted;
(b) a specific antibody of a determined antigen is chosen, then chains each containing a biotinyl radical are fixed covalently to this antibody so as to obtain a polybiotinylated antibody;
(c) the polybiotinylated antibody obtained in (b) is mixed, optionally in the presence of a dilution solvent in particular of the isotonic phosphate buffer (PBS), with an excess of the product obtained in a) in such a way that free avidin sites remain, which are potential receivers of ligands of biotin type, in particular a polybiotinylated antibody or its fragments provided with a biotinylated chain.

**Claims for the following Contracting State : ES**

1. Immunoadsorbent with a low rate of release comprising an antibody and a chromatographic support, characterized in that it contains, on the molecular level:
(a) antibody molecules, each of them bound covalently to several side chains each containing a biotinyl radical, at least one of these said chains being engaged in an affinity bonding with an avidin molecule bound covalently to the said chromatographic support, at least one of these said chains being free and constituting a potential ligand of biotin type for an avidin site of a molecule of avidin type;
(b) avidin molecules bound covalently to the said chromatographic support and containing free sites, potential rereivers of ligands of biotin type.

2. Immunoadsorbent according to claim 1, characterized in addition in that the antibody is monoclonal.

3. Immunoadsorbent according to claim 2, characterized in that the monoclonal antibody is anti-vWF and results from a hybridoma selected after a series of hybridizations of mouse X63 myeloma cells and of BALB/C mouse splenocytes immunized with the FVIII/vWF human complex.

4. Immunoadsorbent according to claim 2, characterized in that the monoclonal antibody is anti-hFSH.

5. Immunoadsorbent according to any one of the previous claims, characterized in that the said chromatographic support is a gel of Sephacryl $^R$ type.

6. Immunoadsorbent according to any one of the previous claims, characterized in that the ratio of the total number of avidin molecules bound to the support to the total number of antibody molecules is about 3 or more.

7. Immunoadsorbent according to any one of the previous claims, characterized in that the average number of biotinylated side chains per antibody molecule is comprised between 3 and 20.

8. Immunoadsorbent according to claim 7, characterized in that the said average number is comprised between 12 and 20.

9. Immunoadsorbent according to any one of the previous claims, characterized in that the amount of polybiotinylated antibody is comprised between 0.1 and 5 mg per cm$^3$ of immunoadsorbent.

10. Process for obtaining an immunoadsorbent with a low rate of release comprising an antibody and a chromatographic support containing, on the molecular level:

(a) antibody molecules, each of them bound covalently to several side chains each containing a biotinyl radical, at least one of these said chains being' engaged in an affinity bonding with an avidin molecule bound covalently to the said chromatographic support, at least one of these said chains being free and constituting a potential ligand of biotin type for an avidin site of a molecule of avidin type;

(b) avidin molecules bound covalently to the said chromatographic support and containing free sites, potential receivers of ligands of biotin type, in which the following operations are carried out:

(a) avidin molecules are fixed covalently to a chromatographic support using a known. method for preserving free sites, which are potential receivers of ligands of biotin type, for the avidin molecules thus grafted;

(b) a specific antibody of a determined antigen is chosen, then chains each containing a biotinyl radical are fixed covalently to this antibody so as to obtain a polybiotinylated antibody;

(c) the polybiotinylated antibody obtained in (b) is mixed, optionally in the presence of a dilution solvent in particular of the isotonic phosphate buffer (PBS), with an excess of the product obtained in a) in such a way that free avidin sites remain, which are potential receivers of ligands of biotin type, in particular a polybiotinylated antibody or its fragments provided with a biotinylated chain.

11. Process according to claim 10, characterized in that the antibody is monoclonal.

12. Process according to claim 11, characterized in that the monoclonal antibody is anti-vWF and results from a hybridoma selected after a series of hybridizations of mouse X63 myeloma cells and of BALB/C mouse splenocytes immunized with the FVIII/vWF human complex.

13. Process according to claim 11, characterized in that the monoclonal antibody is anti-hFSH.

14. Process according to any one of claims 10 to 13, characterized in that the said chromatographic support is a gel of Sephacryl type.

15. Process according to any one of claims 10 to 14, characterized in that the ratio of the total number of avidin molecules bound to the support to the total number of antibody molecules is about 3 or more.

16. Process according to any one of claims 10 to 15, characterized in that the average number of biotinylated side chains per antibody molecule is comprised between 3 and 20.

17. Process according to claim 16, characterized in that the said average number is comprised between 12 and 20.

18. Process according to any one of claims 10 to 17, characterized in that the amount of polybiotinylated antibody is comprised between 0.1 and 5 mg per $cm^3$ of immunoadsorbent.